# EUROPEAN PATENT SPECIFICATION

(11) **EP 0 573 491 B1**
(45) Date of publication and mention of the grant of the patent: **03.04.2002**
(21) Application number: 92905434.4
(22) Date of filing: 30.12.1991
(51) Int. Cl.: C07K 14/78, A61F 2/28, A61K 38/17

(54) **MINERALIZED COLLAGEN**
MINERALISIERTES KOLLAGEN
COLLAGENE MINERALISE

(43) Date of publication of application: 15.12.1993
(73) Proprietor: NORIAN CORPORATION, Mountain View California 94043 (US)
(72) Inventor: CONSTANTZ, Brent, Richard, Scotts Valley, CA 95066 (US); GUNASEKARAN, Subramanian, Newark, CA 94560 (US)
(74) Representative: Harrison, David Christopher
(86) International application number: US9109533
(87) International publication number: WO9312736

(56) References cited:
- DE-A- 2 022 498
- US-A- 3 443 261
- US-A- 3 968 567
- US-A- 4 780 450
- US-A- 4 880 610

## Description

The field of this invention is mineralized collagen.

Enhancement of the compressive, tensile, flexural and fatigue properties of organic composites, has and continues to be an elusive goal. Since the utilization of organic composites in high performance applications is ever-increasing, so is the need to enhance mechanical behavior of these widely used materials.

By mimicking cell-assembled biological materials with high compressive strengths, similar attractive properties may be engineered into new synthetic composites. Bone is a biological composite with an aver modulus of elasticity of about 20 GN/m², compressive strength of 170-220 MN/m², tensile strength of 180 MN/m² and bending strength of 220-270 MN/m².

Bone differs from other composite materials in that it possesses an orderly intimate combination of a calcium phosphate mineral phase within the collagen biopolymer matrix phase. The calcium phosphate appears to be assembled at gaps in the collagen fibrils to create mineral-polymer composite fibers. These mineralized collagen fibers are bonded together in an orderly manner by further calcium phosphate cementation, producing a self-assembling composite.

Bone is characteristically composed of type I collagen fibrils intimately associated in an orderly manner with calcium phosphate crystals. Minor constituents include an array of macromolecules as well as a series of small molecules associated mainly with the mineral phase. Besides bone, similar composite structures are also found in tooth dentin and cementum, fish scales and mineralized tendons. Although the ultrastructural organizational patterns of these tissues differ from one another and from most bones, they all appear to have many properties in common at the molecular level of organization. For example, during their formation collagen is synthesized, extruded from the cell to form self-assembled fibrils in the extracellular space before mineralization begins.

One feature of bone is the exceedingly small size of the crystals. Bone crystals are certainly among the smallest biologically formed crystals known and, in fact, most crystallographers would intuitively not expect crystals just a few unit cells thick to be stable at all. Therefore, the collagen bone structure has unique characteristics as to its formation, components, and properties.

Since collagen is readily available from a wide variety of natural sources and by mineralizing collagen, unique properties may be achievable, there is substantial interest in providing for methods for producing mineralized collagen having characteristics useful for applications in the biomedical as well as other fields.

### Relevant Literature

The protein composition of bone is described by Delmas et al., Calcif. Tissue Int. 3636:308-316 (1984); Tracy et al. In: Development and Diseases of Cartilage and Bone Matrix. ed. A. Sen and T. Thornhill, Alan R. Liss, NY 127-136, (1987). Glimcher, Phil., Trans. R. Soc. Lond. B304 479-508 (1984) describes mineral phases in bone. See also, Calcified Tissue, ed. David W.L. Hukinz, CRC Press Ins., Boca Raton, FL, 1989, chapters 6 and 7. In DE-A-2022498 (= GB-A-1271763) and US-A-3443261 a collagen with a calcium phosphate attached is made by first adding a calcium-containing compound to link with the collagen and then, after treatments, adding phosphoric acid. In US-A-3968567 solutions of collagen calcium and phosphate are instantaneously mixed to form a gel.

In the present invention, mineralized collagen is achieved by employing dispersed or solubilized collagen, preferably in a basic pH medium and preparing calcium phosphate in situ by the addition of calcium and phosphate-containing solutions over a period of at least one hour. The resulting product has calcium phosphate stably dispersed in an ordered manner associated with the collagen fibrils, with a substantially uniform distribution of calcium phosphate crystals distributed through collagen fibrils. Embodiments of the method employ preparing calcium phosphate in situ in a dispersion of collagen fibrils at high pH, e.g. 10-13. The calcium phosphate is formed in situ by the at least partly simultaneous gradual addition, preferably continuous addition, of a source of soluble calcium and a source of soluble phosphate to the collagen-fibril-containing medium. Other components which are to be incorporated into the crystal lattice will also be present. The resulting product after thorough washing in distilled water has calcium phosphate stably distributed with the fibrils to form a substantially uniform composition of calcium phosphate and collagen. The mineral phase will usually have a Ca:P ratio of 1.2-1.8, hexagonal symmetry and be a member of the hydroxyapatite mineral group: it may be 20 to 40 weight percent of the mineralized collagen.

The collagen may come from mineralized or unmineralized collagen sources, usually unmineralized collagen sources. Thus, the collagen may come from bone, tendons, skin, or the like, preferably collagen which involves a combination of two strands of α2 collagen chains. The collagen may be from a young source, e.g., calf, or a mature source, e.g., cow of 2 or more years. The particular source of the collagen, may be any convenient animal source, mammalian or avian, and may include bovine, porcine, equine, or the like, or chicken, turkey, or other domestic source of collagen. The collagen which is employed will normally be dispersed or solubilized collagen where solubilization is achieved by dispersing the collagen source in a medium at an elevated pH, using at least about pH 8, more usually about pH 11-12, and generally less than about 1 N. Commonly, sodium hydroxide is employed, although other hydroxides may find use, such as other alkali metal hydroxides or ammonium hydroxide.

The concentration of collagen will generally be in the range of about 0.1 to 10 weight percent, more usually from about 1 to 5 weight percent. The collagen medium will generally be at a concentration of the base in the range of about 0.0001 to 0.1 N. The pH is generally maintained during the course of the reaction in the range of about 10-13, preferably about 12.

The phosphate and calcium are added as solutions, generally at a concentration in the range of about 0.001-0.5 M, preferably about 0.025-0.2 M. The volume of the solutions added to the collagen medium will generally increase the collagen medium volume by at least 10 percent, usually at least 25 percent and not more than about 400 percent, generally in the range of about 50 to 150 percent. Thus, the collagen solution will generally not be diluted by more than four-fold.

Besides a source of calcium and phsophate, sources of other ions may be employed, such as carbonate, chloride, fluoride, sodium or ammonium. The presence of carbonate results in a product having the properties of dahlite (carbonated hydroxyapatite), while fluoride provides a product having the properties of fluoridated apatite. The weight % of carbonate will usually not exceed 10, while the weight % of fluoride will usually not exceed 2, preferably in the range of 0 to 1. These ions may be present in conjunction with the calcium and/or phosphate source, so long as the ions are compatible and do not result in precipitation in the reagent solutions. For the most part, the counter ions will be selected so as to be physiologically acceptable and not interfere with the mineralization of the collagen fibrils. For the most part the counter ions for calcium will be chloride, oxide, hydroxide, various calcium phosphates, and the like. For the most part, the counter ions for the phosphate will be alkali metals or ammonium, particularly sodium.

The rate of addition is relatively slow, generally requiring at least about one hour and not more than about 72 hours, generally being in the range of about 2 to 18 hours, more usually in the range of about 4 to 16 hours. For example, with one liter of a collagen dispersion, where about a total of about one liter of reagents is added, the rate of addition will generally be in the range of 20 to 150 ml per hour.

The addition of the reagents can be provided in a stoichiometric ratio, although stoichiometry is not required and variations from stoichiometry of up to about 50 percent, preferably not more than about 25 percent are preferred. Thus, where the stoichiometry of addition is not maintained, one of the components may be exhausted, while addition of the other components continues.

During the course of the reaction, mild agitation is maintained, so as to ensure substantially uniform mixing of the collagen fibrils and the calcium phosphate mineral. Mild temperatures are employed, usually not less than about 4°C and not more than about 40°C, preferably in the range of about 15 to 30°C. The weight ratio of the collagen to calcium phosphate mineral will generally be in the range of about 8:2 to 1:1, more usually about 7:3.

The nature of the calcium phosphate mineral may be varied widely, including calcium hydroxyapatite, calcium hydroxy/fluorapatide, brushite, dahlite, monetite, phosphated calcium carbonate (calcite), octacalcium phosphate, tricalcium phosphate, where the choice of stoichiometry of the calcium and the phosphate, as well as the presence of other ions, will result in the particular composition. Thus, by varying the calcium to phosphate ratio, a range of mineral compositions may be attained. Other non-collagenous proteins or factors, such as BMP-2, calcitonin, etc. may be included in the solubilized or dispersed collagen slurry, prior to Ca and PO₄ addition. The amount of such additives will generally be in the range of about 0.0001 to 2 weight % based on collagen. The added protein may combine with the mineral as it forms on the collagen, binding the added protein to the collagen.

After completion of the addition, agitation, e.g., stirring, will normally be continued, usually at least about 1 h, more usually about 2 h and agitation may continue even more. The amount of continued agitation is not critical to the preparation of the product.

Upon completion of the reaction, the product may be treated in a variety of ways. The product may be washed repeatedly to remove any unbound minerals or other components of the medium, as well as provide a more neutral pH. Washing may be readily accomplished with water, saline, mild acids or the like. The product may be stored in solution, may be lyophilized, filtered and dried, or the like.

The amount of collagen present in the final product will generally be from about 80% to 30%. The bulk density of the final product will be in the range of about 0.01 to 0.5. The mineralized collagen will retain the calcium phosphate mineral under standard temperature and humidity. The TEM/SEM and FTIR indicate the presence of both collagen and calcium phosphate mineral which is more likely a carbonated apatite.

The subject compositions may be further treated in a variety of ways. The subject compositions may be cross-linked using a variety of cross-linking agents, such as formaldehyde, glutaraldehyde, chromium salts, di-isocyanates or the like. The subject compositions may be combined with other compositions, such as hydroxyapatite, as gravel, slurry, powder, etc. These combinations may find use for different hard tissue implantations.

The subject composition may find application in a wide variety of bio-medical uses, by themselves or in combination. The subject composition may be used for the production of prosthetic devices, bone filler, hemostatic agent for bone-bleeding, etc.

The mineralized collagen has application as an osteoconductive bone grafting material for non-union fractures, periodontal defect filler and bony defect filler. By combining the subject composition with an osteoinductive material, such as autologous aspirated bone marrow, bone morphogenic protein, calcitonin or other growth factors, bone induction and growth may be further augmented. The subject compositions find application in soft tissue augmentation, such as urinary sphincter augmentation, dermal wrinkle augmentation and burn tissue repair. In addition, the subject compositions may be used as a hemostatic agent, particularly for hard tissue bleeding.

The following examples are offered by way of illustration and not by way of limitation.

### EXPERIMENTAL

Example 1: Reaction solutions were prepared of 0.06M sodium phosphate (tri-basic) and 0.1M calcium chloride. Bovine tendon collagen (2.15 g) was solubilized in one liter of sodium hydroxide, pH 12. The reagent solutions were added simultaneously over a period of five hours and ten minutes. The mixture was kept in the refrigerator undisturbed overnight. The mineralized collagen product was centrifuged and then washed three times with deionized water followed by centrifugation. (U-0071). The weight ratio of collagen to mineral was 3:7,

The above experiment was repeated except that the collagen source was different. Avian bone collagen from Medimatrix was employed. (U-0074).

The above samples were lyophilized and viewed under SEM (Scanning Electron Microscope). While no banding structure was observed, a fibrillar structure was seen. EDS (Electron Dispersive Spectrometry) showed a similar calcium to phosphorous ratio as seen in hydroxyapatite, with the calcium distributed uniformly throughout the sample. This is evident with the SEM elemental analyses. When a portion of the sample U-0071 which was not lyophilized was suspended in 500 ml of deionized water, and blended for one minute, followed by pouring into a 500 ml graduated cylinder and kept undisturbed in a refrigerator, at four hours no separation of phases was observed.

The lyophilized powders were measured for bulk (apparent) density, where U-0071 was found to be 0.04, while U-0074 was found to be 0.01.

Example 2: Avian bone collagen (11.6 g) was dissolved in 1 liter of dilute NaOH solution (pH 12). Reagent solutions of sodium tri-basic phosphate and calcium chloride at 0.06 M and 0.1 M, respectively were prepared. The concentration of the collagen in the solution was approximately 1% (w/v). The addition of the reagents required 72 hours and provided a ratio of 70% collagen: 30% hydroxyapatite by weight. At the end of this time, the reaction was stopped and the sample stored in a refrigerator. The sample was washed three times in distilled water with centrifugation and then stored in a freezer (U-0084).

The above results demonstrate that one can prepare stable mineralized collagen for a variety of uses. The calcium phosphate mineral is stably distributed in the collagen to provide a product having desirable physical properties and mimicks bone. It is mineralogically, biochemically, and ultrastructurally nearly identical to mineralized bone. Spacial arrangements of crystals of hydroxyapatite and collagen, shield the crystals from being perceived by the immune system as foreign bodies, analogous to the way that ceramic gravels mixed with collagen are used.

The invention now being fully described, it will be apparent to one of ordinary skill in the art that many changes and modifications can be made thereto.

## Claims

1. A method of mineralizing collagen with a calcium phosphate mineral, said method comprising:
preparing said calcium phosphate mineral in situ in the presence of a dispersion of solubilized or dispersed collagen fibrils in basic pH aqueous medium by adding solutions of soluble calcium and soluble phosphate to said dispersion over a period of at least one hour; and
collecting the mineralized collagen.

2. A method according to claim 1 wherein the addition is in the correct stoichiometry for said calcium phosphate mineral.

3. A method according to claim 1 or claim 2, wherein said calcium phosphate mineral has a Ca:P ratio of 1.2-1.8, has hexagonal symmetry and is a member of the hydroxyapatite mineral group.

4. A method according to claim 1, wherein said dispersion is a solution.

5. A method according to claim 1, wherein said pH is in the range of 10 to 13 and is maintained during said preparing.

6. A method according to claim 5, wherein the pH is about 12.

7. A method according to claim 1, wherein said source of calcium is calcium chloride and said source of phosphate is sodium or ammonium phosphate.

8. A method according to claim 1, wherein the addition of said source of calcium and said source of phosphate during said preparing is at other than in stoichiometric proportion.

9. A method according to claim 1, wherein said collagen is bovine collagen.

10. A method according to claim 1, wherein a non-collagenous protein moiety is added to the dispersion prior to the adding of the said solutions.

11. A method according to any one of the preceding claims wherein the volume of said dispersion is diluted less than about four-fold by the addition.

12. A method according to claim 11 wherein the amount of calcium phosphate mineral is 20 to 40 weight percent of said mineralized collagen.

13. A formed object composed at least in part of a mineralized collagen prepared according to the method of claim 1.

14. A formed object according to claim 13 which additionally includes an osteoinductive material.

15. A method according to claim 1, wherein said calcium phosphate mineral also contains carbonate.

16. A method according to claim 15, wherein said carbonate is present in said mineral in an amount up to 10 wt. %.

## Patentansprüche

1. Verfahren zum Mineralisieren von Collagen mit einem Kalziumphosphat-Mineral, wobei das Verfahren Folgendes umfasst:
das Herstellen des Calciumphosphat-Minerals in situ in Gegenwart einer Dispersion von solubilisierten oder dispergierten Collagen-Fibrillen in wässrigem Medium mit basischem pH durch Zugabe von Lösungen von löslichem Kalzium und löslichem Phosphat zu der Dispersion über einen Zeitraum von zumindest 1 h; und
das Gewinnen des mineralisierten Collagens.

2. Verfahren nach Anspruch 1, worin die Zugabe mit der richtigen Stöchiometrie für das Kalziumphosphat-Mineral erfolgt.

3. Verfahren nach Anspruch 1 oder 2, worin das Kalziumphosphat-Mineral ein Verhältnis Ca : P von 1,2-1,8 aufweist, hexagonale Symmetrie aufweist und ein Element der Hydroxyapatit-Mineralgruppe ist.

4. Verfahren nach Anspruch 1, worin die Dispersion eine Lösung ist.

5. Verfahren nach Anspruch 1, worin der pH im Bereich von 10 bis 13 liegt und während der Herstellung beibehalten wird.

6. Verfahren nach Anspruch 5, worin der pH etwa 12 beträgt.

7. Verfahren nach Anspruch 1, worin die Calcium-Quelle Calciumchlorid ist und die Phosphat-Quelle Natrium- oder Ammoniumphosphat ist.

8. Verfahren nach Anspruch 1, worin die Zugabe der Calcium-Quelle und der Phosphat-Quelle während der Herstellung nicht im stöchiometrischen Verhältnis erfolgt.

9. Verfahren nach Anspruch 1, worin das Collagen Rinder-Collagen ist.

10. Verfahren nach Anspruch 1, worin eine nicht-collagenartige Proteingruppe der Dispersion vor der Zugabe der Lösungen zugesetzt wird.

11. Verfahren nach einem der vorangegangenen Ansprüche, worin das Volumen der Dispersion durch die Zugabe auf weniger als etwa das Vierfache verdünnt wird.

12. Verfahren nach Anspruch 11, worin die Menge an Kalziumphosphat-Mineral 20 bis 40 Gew.-% des mineralisierten Collagens beträgt.

13. Geformter Gegenstand, der zumindest teilweise aus einem nach dem Verfahren nach Anspruch 1 hergestellten mineralisierten Collagen besteht.

14. Geformter Gegenstand nach Anspruch 13, der zusätzlich ein osteoinduktives Material umfasst.

15. Verfahren nach Anspruch 1, worin das Calciumphosphat-Mineral auch Carbonat enthält.

16. Verfahren nach Anspruch 15, worin das Carbonat im Mineral in einer Menge von bis zu 10 Gew.-% enthalten ist.

## Revendications

1. Méthode de minéralisation de collagène avec un minéral de phosphate de calcium, ladite méthode comprenant:
la préparation dudit minéral de phosphate de calcium in situ en présence d'une dispersion de fibrilles de collagène solubilisées ou dispersées dans un milieu aqueux à pH basique par addition de solutions, de calcium soluble et de phosphate soluble à ladite dispersion sur une période d'au moins une heure; et
la collecte du collagène minéralisé.

2. Méthode selon la revendication 1, où l'addition est à la stoechiométrie correcte pour ledit minéral de phosphate de calcium.

3. Méthode selon la revendication 1 ou 2, où ledit minéral de phosphate de calcium a un rapport Ca:P de 1,2-1,8, a une symétrie hexagonale et est un membre du groupe minéral d'hydroxyapatite.

4. Méthode selon la revendication 1, où ladite dispersion est une solution.

5. Méthode selon la revendication 1, où ledit pH est dans la gamme de 10 à 13 et est maintenu pendant ladite préparation.

6. Méthode selon la revendication 5, où le pH est d'environ 12.

7. Méthode selon la revendication 1, où ladite source de calcium est le chlorure de calcium et ladite source de phosphate est le phosphate de sodium ou d'ammonium.

8. Méthode selon la revendication 1, où l'addition de ladite source de calcium et de ladite source de phosphate pendant ladite préparation et en une proportion autre que stoechiométrique.

9. Méthode selon la revendication 1, où ledit collagène est du collagène de bovin.

10. Méthode selon la revendication 1, où une fraction de protéine non collagène est ajoutée à la dispersion avant addition desdites solutions.

11. Méthode selon l'une quelconque des revendications précédentes, où le volume de ladite dispersion est dilué à moins d'environ quatre fois par l'addition.

12. Méthode selon la revendication 11, où la quantité du minéral de phosphate de calcium est de 20 à 40 pour cent en poids dudit collagène minéralisé.

13. Objet formé composé au moins en partie d'un collagène minéralisé préparé selon la méthode de la revendication 1.

14. Objet formé selon la revendication 13 qui contient additionnellement un matériau ostéoinducteur.

15. Méthode selon la revendication 1, où ledit minéral de phosphate de calcium contient également du carbonate.

16. Méthode selon la revendication 15, où ledit carbonate est présent dans ledit minéral en une quantité jusqu'à 10% en poids.
